# EUROPÄISCHE PATENTSCHRIFT

(11) **EP 1 907 118 B1**
(45) Veröffentlichungstag und Bekanntmachung des Hinweises auf die Patenterteilung: **05.11.2008**
(21) Anmeldenummer: 06754276.1
(22) Anmeldetag: 10.06.2006
(51) Int. Cl.: B01J 23/75, C07C 209/02, C08G 65/325

(54) **VERFAHREN ZUR HERSTELLUNG VON POLYETHERAMINEN**
POLYETHERAMINE PRODUCTION METHOD
PROCEDE DE PRODUCTION DE POLYETHERAMINES

(30) Priorität: 28.06.2005 DE 102005029932
(43) Veröffentlichungstag der Anmeldung: 09.04.2008
(73) Patentinhaber: Clariant Produkte (Deutschland) GmbH, 65929 Frankfurt am Main (DE)
(72) Erfinder: BÜHRING, Dirk, 84489 Burghausen (DE); GALLAS, Andreas, 84567 Erlbach (DE); GLOS, Martin, 46325 Borken (DE); RAAB, Klaus, 84508 Burgkirchen (DE); SCHERL, Franz-Xaver, 84508 Burgkirchen (DE); WACHSEN, Olaf, 84518 Garching (DE)
(74) Vertreter: Mikulecky, Klaus
(86) Internationale Anmeldenummer: PCT/EP2006/005577
(87) Internationale Veröffentlichungsnummer: WO 2007/000236

(56) Entgegenhaltungen:
- EP-A2- 0 343 486
- EP-A2- 0 436 235

## Beschreibung

Die vorliegende Erfindung betrifft ein Verfahren zur Herstellung von Polyetheraminen unter Verwendung von Cobalt-Katalysatoren.

Polyetheramine enthalten mindestens eine Polyalkylenglykolgruppe, die deren Eigenschaften maßgeblich beeinflusst. Bei hohem Anteil an Polyethylenglykoleinheiten erhält man wasserlösliche und bei hohem Anteil von Polypropylenglykoleinheiten wasserunlösliche Polyetheramine. Außerdem lassen sich durch die Veränderung der Molmassen der Polyalkylenglykole Schmelzpunkt und Viskosität der Polyetheramine beeinflussen. Durch die Auswahl geeigneter Startalkohole für die Alkoxylierung lassen sich tensidische Eigenschaften erzielen. Außerdem kann man mit mehrwertigen Alkoholen verzweigte Polyetheramine aufbauen, die dann nach der Aminolyse zu mehrwertigen Aminen führen. Damit ergeben sich vielfältige Möglichkeiten durch die Wahl geeigneter Polyalkylenglykole die Eigenschaften eines darauf basierenden Polyetheramins gezielt zu beeinflussen.

Der Stand der Technik offenbart verschiedene Verfahren zur Herstellung von Polyetheraminen.

DE-A-16 43 426 beschreibt ein Verfahren zur Herstellung von . Polyoxyalkylenaminen, ausgehend von den entsprechenden Alkoholen unter Verwendung eines Nickel-Kupfer-Chrom-Katalysators, der 60-85 Mol-% Nickel, 14-37 Mol-% Kupfer und 1-5 Mol-% Chrom enthält.

US-4 618 717 beschreibt ein Verfahren zur Herstellung von primären Aminen, ausgehend von Oxyethylenglykol-Monoalkylethern unter Verwendung eines Katalysators, der 50-90 Gew.-% Nickel, 10-15 Gew.-% Kupfer und 0,5-5 Gew.-% der Elemente Chrom, Eisen, Titan, Thorium, Zirkon, Mangan, Magnesium oder Zink enthält.

US-5 352 835 beschreibt einen Trägerkatalysator zur Aminolyse, der zur Umsetzung von Polyoxyalkylenglykolen zu den entsprechenden Aminen verwendet wird. Hierbei besteht der Katalysator aus 15-30 Gew.-% Nickel, 3-20 Gew.-% Kupfer, 0,5-1 Gew.-% Molybdän und mindestens 50 Gew.-% γ-Aluminiumoxid, welches als Trägermaterial dient.

US-4 766 245 beschreibt ein Verfahren zur Herstellung von Polyoxyalkylenpolyaminen ausgehend von den entsprechenden Alkoholen unter Verwendung eines Raney-Nickel/Aluminium-Katalysators der zu 60-75 % aus Nickel und zu 40-25 % aus Aluminium besteht.

DE-A-36 08 716 beschreibt ein Verfahren zur Herstellung von Polyoxyalkylenpolyaminen, ausgehend von den entsprechenden Alkoholen unter Verwendung eines Raney-Nickel- oder Raney-Nickel/Aluminium-Katalysators, der zusätzlich noch 0,2-5% Molybdän enthält.

US-5 003 107 beschreibt ein Verfahren zur reduktiven Aminierung von Polyoxytetramethylenglykolen unter Verwendung eines Katalysators, der 70-75 Gew.-% Nickel, 20-25 Gew.-% Kupfer, 0,5-5 Gew.-% Chrom und 1-5 Gew.-% Molybdän enthält.

DE-A-44 28 004 beschreibt ein Verfahren zur Herstellung von Aminen ausgehend von Alkoholen, bei dem der Katalysator 20-85 % Zr, 1-30 % Cu, 30-70 % Ni, 0,1-5 % Mo, 0-10 % Aluminium und/oder Mangan, jeweils als Oxid berechnet, enthält.

US-A-2003/0139289 beschreibt ein verbessertes Verfahren zur Herstellung von Aminen ausgehend von Alkoholen, Aldehyden oder Ketonen mittels eines Katalysators, der neben Nickel, Kupfer und Chrom noch Zinn enthält.

DE-A-19 53 263 beschreibt ein Verfahren zur Herstellung von Aminen ausgehend von Alkoholen mittels eines Katalysators. Der Metallanteil des Katalysators besteht zu 70 bis 95 % aus Co und Ni und zu 5 bis 30% aus Kupfer. Hierbei reicht das Gewichtsverhältnis von Co zu Ni von 4:1 bis 1:4.

DE-A-102 11 101 beschreibt ein Verfahren zur Herstellung von Aminen ausgehend von Alkoholen oder Aldehyden, bei dem der Katalysator 22-40 % Zr, 1-30 % Cu, 15-50 % Ni, 15-50 % Co, jeweils als Oxid berechnet, enthält.

DE-A-15 70 542 offenbart ein Verfahren zur Herstellung von Polyetheraminen, dadurch gekennzeichnet, dass man Polyether der Formel I oder II

HO-R-(OR)ₙ-OH (I)

Z[(OR)ₙ-OH]ₘ (II)

worin R einen aliphatischen Rest mit 2-4 C-Atomen bedeutet, Z für einen zwei- bis sechswertigen aliphatischen, araliphatischen, aromatischen oder alicyclischen Rest steht, der ein- oder mehrfach durch Ether- oder Aminogruppen, Carbonamid-, Urethan- oder Harnstoffgruppen unterbrochen sein kann, n ganze Zahlen zwischen 1 und 50 und m = 2 bis 6 je nach der Wertigkeit von Z darstellt, in Gegenwart von Wasser und Wasserstoff an einem hauptsächlich aus einem Element der 8. Nebengruppe bestehenden Hydrierungs-Dehydrierungskontakt mit Ammoniak bei 200-280°C, vorzugsweise 220-250°C, unter Druck umsetzt.

Überraschenderweise wurde nun gefunden, dass Polyetheramine bzw. Polyoxyalkylenamine aus den entsprechenden Alkoholen und Ammoniak bzw. Aminen (in Gegenwart von Wasserstoff) hergestellt werden können, indem man einen Katalysator verwendet, dessen Metallanteil zu mindestens 80 Gew.-% aus Cobalt besteht.

Es können hierbei sowohl Trägerkatalysatoren, die außer dem katalytisch aktiven Metallanteil noch Trägermaterial enthalten, als auch Metallkatalysatoren ohne zusätzliches Trägermaterial, wie beispielsweise die sogenannten "Raney-Typen" verwendet werden.

Das Verfahren ist sowohl zur kontinuierlichen als auch zur diskontinuierlichen Herstellung von Polyetheraminen geeignet.

Gegenstand der Erfindung ist somit ein Verfahren zur Herstellung von Aminen der Formel 1 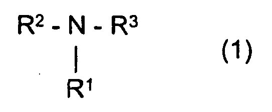 worin R² einen organischen Rest darstellt, der zwischen 2 und 600 Alkoxygruppen umfasst, und R¹ und R³ gleich oder verschieden sind und Wasserstoff oder einen organischen Rest mit 1 bis 400 Kohlenstoffatomen darstellen, indem man eine Verbindung der Formel 2 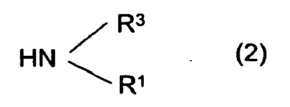 mit einer Verbindung der Formel 3

R²-OH

in Gegenwart von Wasserstoff mit einem Katalysator in Verbindung bringt, der metallhaltig ist und dessen Metallgehalt zu mindestens 80 Gew.-% aus Cobalt besteht.

Der für das erfindungsgemäße Verfahren verwendete Katalysator enthält vorzugsweise 85, insbesondere 90, speziell 95 Gew.-% Cobalt, bezogen auf den Gesamtmetallgehalt des Katalysators. Der Katalysator kann neben Cobalt noch Nickel, Kupfer, Chrom, Eisen, Titan, Thorium, Zirkonium, Mangan, Magnesium, Molybdän, Aluminium, Zink und/oder Zinn enthalten.

Der Katalysator kann ein geträgerter oder ein ungeträgerter Katalysator sein.

Ist der Katalysator ungeträgert, so handelt es sich vorzugsweise um einen Katalysator vom Raney-Typ.

Ist der Katalysator geträgert, so können die Trägerteilchen des Katalysators eine beliebige geometrische Form haben, beispielsweise die Form von Kugeln, Tabletten oder Zylindern in regelmäßiger oder unregelmäßiger Ausführung. Die Abmessung der Trägerteilchen liegt im Allgemeinen zwischen 1 und 8 mm. Bevorzugt ist die Kugelform, zum Beispiel Kugeln mit einem Durchmesser von 4 bis 8 mm. Die Trägerteilchen werden im Allgemeinen Pellets genannt.

Als Träger eignen sich die bekannten inerten Trägermaterialien wie Kieselsäure, Aluminiumoxid, Alumosilikate, Silikate, Titanoxid, Zirkonoxid, Titanate, Siliciumcarbid und Kohle. Besonders geeignet sind Träger dieser Art mit einer spezifischen Oberfläche von 50 bis 300 m²/g (gemessen nach der BET-Methode) und einem mittleren Porenradius von 50 bis 2000 Å (gemessen mit Quecksilber-Porosimetrie), vor allem Kieselsäure (SiO₂) und SiO₂-Al₂O3-Gemische.

Bei Trägerkatalysatoren kann der Metallgehalt zwischen 1 und 99% liegen, bevorzugt im Bereich von 5 bis 70%.

R² enthält 2 bis 600 Alkoxygruppen. Unter Alkoxygruppen wird vorliegend eine Einheit der Formel -(AO)- verstanden, worin A für eine C₂- bis C₄-Alkylengruppe steht. 2 bis 600 Alkoxygruppen bedeuten damit eine Struktureinheit der Formel -(AO)ₙ- mit n = 2 bis 600.

In der durch (A-O)ₙ wiedergegebenen Alkoxykette bedeutet A vorzugsweise einen Ethylen- oder Propylenrest, insbesondere einen Ethylenrest. Die Gesamtzahl von Alkoxyeinheiten liegt vorzugsweise zwischen 5 und 300, insbesondere zwischen 8 und 200. Bei der Alkoxykette kann es sich um eine Blockpolymerkette handeln, die alternierende Blöcke verschiedener Alkoxyeinheiten, vorzugsweise Ethoxy- und Propoxyeinheiten, aufweist. Es kann sich dabei auch um eine Kette mit statistischer Abfolge der Alkoxyeinheiten, oder ein Homopolymer handeln.

In einer bevorzugten Ausführungsform steht -(A-O)ₙ- für eine Alkoxykette der Formel 4

-(CH₂-CH(CH₃)-O)ₐ-(CH₂-CH₂-O)_{b}-(CH₂-CH(CH₃)-O)_{c}- (4)

worin
a eine Zahl von 0 bis 300, vorzugsweise 0 bis 80
b eine Zahl von 3 bis 300, vorzugsweise 3 bis 200
c eine Zahl von 0 bis 300, vorzugsweise 0 bis 80 bedeuten.

R¹ und R³ sind gleich oder verschieden und stehen für Wasserstoff oder einen organischen Rest mit 1 bis 400 Kohlenstoffatomen. R¹ und R³ können neben Kohlenstoff und Wasserstoff auch Heteroatome wie Sauerstoff, Stickstoff, Phosphor oder Schwefel enthalten.

In einer bevorzugten Ausführungsform sind R¹ und R³ Wasserstoff, ein Alkylrest mit 1 bis 50 Kohlenstoffatomen, ein Alkenylrest mit 2 bis 50 Kohlenstoffatomen, ein Arylrest mit 6 bis 50 Kohlenstoffatomen, oder ein Alkylarylrest mit 7 bis 50 Kohlenstoffatomen.

In einer weiteren bevorzugten Ausführungsform entsprechen R¹ und R³ der gleichen Definition wie R².

In einer weiteren bevorzugten Ausführungsform enthalten R¹ und R³ Aminogruppen. Vorzugsweise entsprechen R¹ und R³ dann der Formel 5 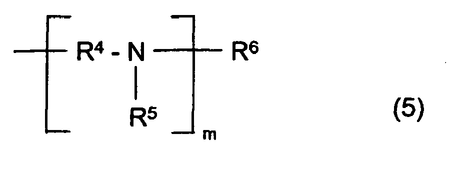 worin R⁴ eine zweiwertige Kohlenwasserstoffgruppe mit 1 bis 50 Kohlenstoffatomen und R⁵ und R⁶ jeweils Wasserstoff oder eine einwertige Kohlenwasserstoffgruppe mit 1 bis 50 Kohlenstoffatomen sein können, wobei jedes von R⁴, R⁵ und R⁶ 1 bis 200 Alkoxygruppen umfassen kann und auch Heteroatome wie Sauerstoff, Stickstoff, Phosphor oder Schwefel enthalten können (im Grunde wie R³), und m für eine Zahl von 1 bis 10 steht.

Wenn R² für alkoxyliertes Methanol steht, so kann das Produkt des erfindungsgemäßen Verfahrens beispielsweise folgende Strukturen aufweisen:

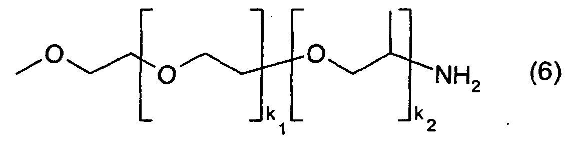

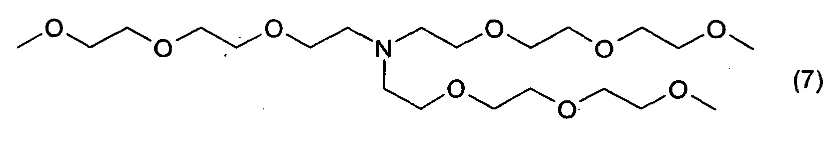

Im Falle eines alkoxylierten Alkyl-Alkohols ergibt sich folgende Struktur:

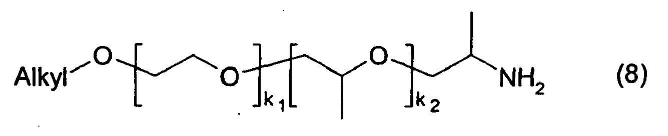

Bei Verwendung von Polyalkylenglykolen (Diolen) kommt man zu folgenden Strukturen: 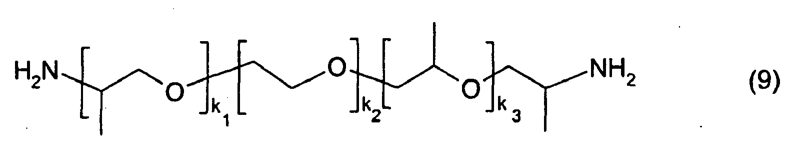 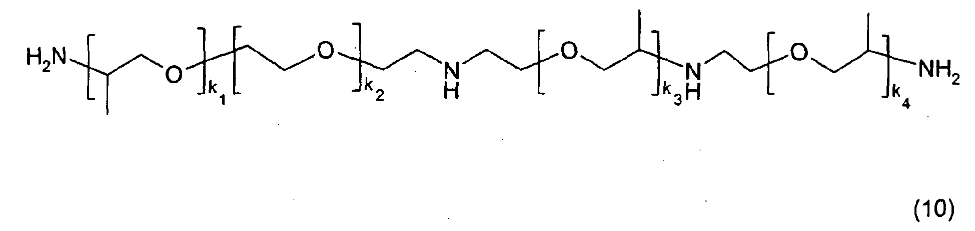 worin k₁, k₂, k₃ und k₄ ganze Zahlen darstellen, die in der Summe bis zu 600
ergeben.

In einer weiteren bevorzugten Ausführungsform steht das Amin der Formel (1) für ein Polyamin der Formel 11

R⁷(NR⁹R⁸)ₙ (11)

worin R⁷ für einen n-wertigen organischen Rest mit 2 bis 400 Kohlenstoffatomen, der auch Heteroatome wie Sauerstoff, Stickstoff, Phosphor oder Schwefel enthalten kann, R⁸ und R⁹ für einen Rest wie R³, und n für eine ganze Zahl von 2 bis 20 stehen.

Wenn R⁷ für alkoxyliertes Glycerin steht, so kann das Produkt des erfindungsgemäßen Verfahrens beispielsweise folgende Struktur aufweisen: 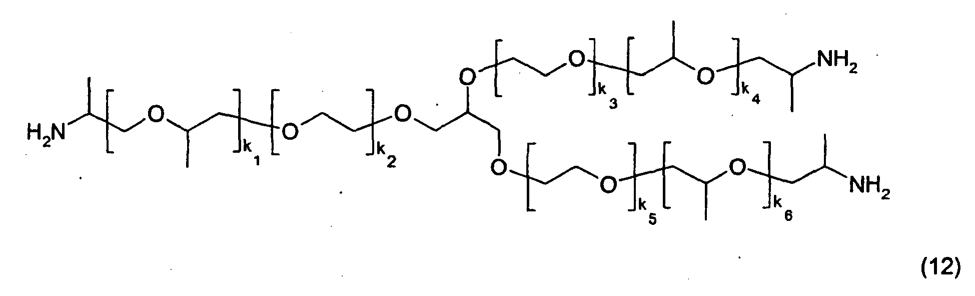 worin die Indices kₙ ganze Zahlen sind, die in der Summe bis zu 600 ergeben.

Wenn R⁷ für alkoxyliertes Octylamin steht, so kann das Produkt des erfindungsgemäßen Verfahrens beispielsweise folgende Struktur aufweisen: 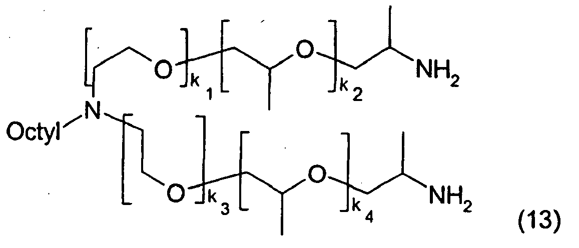 worin die Indices kₙ ganze Zahlen sind, die in der Summe bis zu 600 ergeben.

Erfindungsgemäße Polyetheramine sind ein- oder mehrwertige Amine, die verzweigt, unverzweigt oder cyclisch, gesättigt oder ungesättigt sein können. Solche Amine sind beispielsweise
- einwertige Amine, wie beispielsweise
   Alkylpolyalkylenglykolamine wie z.B. Methyltriethylenglykolamin, Bis(methyltriethylenglykol)amin, Butyltriethylenglykolamin, Laurylpolypropylenglykolamin, Methyltripropylenglykolamin, Phenolpolypropylenglykolamin, iso-Tridecylpolypropylenglykolamin, Bis(methyltripropylenglykol)amin, N-Methyl-Methylpolypropylenglykolamin, Methylpolypropylenglykolamin, Bis(methylpolypropylenglykol)amin, Tris(methyldiglykol)amin, Methylpolyalkylenglykolamin mit statistischer oder blockartiger Verteilung der Ethylen- und Propylenglykol-Einheiten,
- zweiwertige Amine, wie beispielsweise
   Triethylenglykoldiamin, Tripropylenglykoldiamin, Polyethylenglykoldiamine, Polypropylenglykoldiamin, Polylalkylenglykoldiamin mit statistischer oder blockartiger Verteilung der Ethylen- und Propylenglykol-Einheiten, Butandiolpolyalkylenglykoldiamin, Resorcinpolyalkylenglykoldiamin,
- dreiwertige Amine, wie beispielsweise
   Glycerinpolyalkylenglykoltriamin mit statistischer oder blockartiger Verteilung der Ethylen- und Propylenglykol-Einheiten, Bis(triethylenglykolamin)amin, Bis(polyalkylenglykolamin)amin,
- vierwertige Amine, wie beispielsweise
   Pentaerythrolpolyalkylenglykoltetraamin mit statistischer oder blockartiger Verteilung der Ethylen- und Propylenglykol-Einheiten, N,N'-Bis(polypropylenglykolamin)-polyalkylenglykoldiamin.

Als Aminierungsmittel bei der reduktiven Aminierung der Polyetheralkohole zu den Aminen nach dem erfindungsgemäßen Verfahren können sowohl Ammoniak als auch primäre oder sekundäre, aliphatische oder cycloaliphatische oder aromatische Amine eingesetzt werden.

Bei der Verwendung von Ammoniak als Aminierungsmittel wird zunächst ein primäres Amin gebildet. Bei entsprechenden Reaktionsbedingungen (Druck, Temperatur, Reaktionszeit) kann dies als Produkt isoliert werden, oder die Reaktion wird weitergeführt, so dass das gebildete primäre Amin mit weiterem Alkohol zum entsprechenden sekundären oder auch tertiären Amin weiterreagiert.

Neben Ammoniak können beispielsweise folgende Amine als Aminierungsmittel zum Einsatz kommen: Methylamin, Dimethylamin, Ethylamin, Diethylamin, n-Propylamin, Di-n-Propylamin, iso-Propylamin, Di-iso-Propylamin, Hexylamin, Cyclohexylamin, Anilin, Toluidin, Piperidin, Morpholin.

Das Aminierungsmittel kann bezüglich der zu aminierenden Hydroxyl-Gruppe in stöchiometrischen, unter- oder überstöchiometrischen Mengen eingesetzt werden. Ebenso kann die zu verwendende Wasserstoffmenge in einem weiten Bereich, von unter- bis überstöchiometrisch, variiert werden.

Das erfindungsgemäße Verfahren wird vorzugsweise bei Temperaturen von 50 bis 250°C, insbesondere bei Temperaturen von 140°C bis 200°C durchgeführt.

Das erfindungsgemäße Verfahren wird vorzugsweise bei Drucken von 1 bar bis 300 bar, insbesondere bei Drucken von 30 bar bis 200 bar durchgeführt.

Die zu verwendende Katalysatormenge liegt vorzugsweise im Bereich von 0,5 bis größer 90 Gew.-%, insbesondere 1 bis 80 Gew.-%, speziell 2 bis 70 Gew.-%, bezogen auf den verwendeten Alkohol. Mengen über 70 Gew.-% werden insbesondere bei kontinuierlichen Verfahren verwendet.

Die Aminierung kann kontinuierlich oder diskontinuierlich durchgeführt werden. Bei beiden Verfahren können die gasförmigen oder zum Teil auch überkritischen Reaktionskomponenten (Aminierungsmittel, Wasserstoff und eventuell Inertgase) im Kreis gefahren werden.

Das sich während der Reaktion bildende Wasser kann entweder im Reaktionsgemisch verbleiben oder auch gegebenenfalls entfernt werden, falls ansonsten die gewünschte Umsetzung hinsichtlich Ausbeute und/oder Selektivität leidet.

Die Aminierung wird bevorzugt ohne Lösungsmittel durchgeführt. Es können aber auch Lösungsmittel verwendet werden.

Der erhaltene Reaktionsaustrag wird von überschüssigem Aminierungsmittel, Wasserstoff und gegebenenfalls Inertgasen befreit und das Amin je nach Bedarf entsprechend weiter aufgereinigt. Die entfernten Reaktionskomponenten können, eventuell nach entsprechender Aufarbeitung, dem Aminierungsprozess wieder zugeführt werden.

### Beispiele

### Beispiel 1

In einen Schüttelautoklav mit 4,5 I Volumen wurden 1,5 kg Methylpolyalkylenglykol mit einer mittleren Molmasse von 2000 g/mol, 300 g eines Trägerkatalysators, dessen Metallanteil zu mehr als 90 % aus Cobalt bestand, und 800 ml Ammoniak (flüssig) dosiert. Anschließend wurden 10 bar Wasserstoff aufgepresst und der Autoklav verschlossen. Das Gemisch wurde unter Schütteln für 8 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 85 % zum entsprechenden Amin ermittelt werden.

### Beispiel 2

In einen Rührautoklav mit 2 I Volumen wurden 1 kg Methylpolyalkylenglykol mit einer mittleren Molmasse von 2000 g/mol, 200 g eines Trägerkatalysators, dessen Metallanteil zu mehr als 90 % aus Cobalt bestand, und 540 ml Ammoniak (flüssig) dosiert. Anschließend wurden 30 bar Wasserstoff aufgepresst und der Autoklav verschlossen. Das Gemisch wurde unter Rühren für 7 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 95 % zum entsprechenden Amin ermittelt werden.

### Beispiel 3

In einen Rührautoklav mit 2 I Volumen wurden 1 kg Methylpolyalkylenglykol mit einer mittleren Molmasse von 2000 g/mol, 180 g Cobalt-Katalysators vom Raney-Typ und 540 ml Ammoniak (flüssig) dosiert. Anschließend wurden 10 bar Wasserstoff aufgepresst und der Autoklav verschlossen. Das Gemisch wurde unter Rühren für 6 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 95% zum entsprechenden Amin ermittelt werden.

### Beispiel 4

In einen Schüttelautoklav mit 4,5 l Volumen wurden 750 g Polyalkylenglykol bestehend aus Ethylen- und Propylenglykoleinheiten mit einer mittleren Molmasse von 600 g/mol, 150 g eines Trägerkatalysators, dessen Metallanteil zu mehr als 90 % aus Cobalt bestand, und 800 ml Ammoniak (flüssig) dosiert. Anschließend wurden 10 bar Wasserstoff aufgepresst und der Autoklav verschlossen. Das Gemisch wurde unter Schütteln für 6 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 95 % zum entsprechenden Diamin ermittelt werden.

### Beispiel 5

In einen Rührautoklav mit 2 I Volumen wurden 1 kg Polyalkylenglykol bestehend aus Ethylen- und Propylenglykoleinheiten mit einer mittleren Molmasse von 4000 g/mol, 200 g eines Trägerkatalysators, dessen Metallanteil zu mehr als 80 % aus Cobalt bestand, und 540 ml Ammoniak (flüssig) dosiert. Anschließend wurden 10 bar Wasserstoff aufgepresst und der Autoklav verschlossen. Das Gemisch wurde unter Rühren für 5 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von größer 95 % zum entsprechenden Amin ermittelt werden.

### Beispiel 6

In einen Schüttelautoklav mit 4,5 I Volumen wurden 1,5 kg Methylpolyalkylenglykol mit einer mittleren Molmasse von 2000 g/mol, 100 g Cobalt-Katalysator vom Raney-Typ und 800 ml Ammoniak (flüssig) dosiert. Anschließend wurden 10 bar Wasserstoff aufgepresst und der Autoklav verschlossen. Das Gemisch wurde unter Schütteln für 12 Stunden auf 190°C erwärmt, wobei durch weiteres Aufpressen von Wasserstoff ein Druck von 180 bar gehalten wurde. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 85 % zum entsprechenden Amin ermittelt werden.

### Beispiel 7

In einen Rührautoklav mit 2 I Volumen wurden 1 kg Fettalkoholoxpropylat mit einer mittleren Molmasse von 310 g/mol, 200 g eines Trägerkatalysators, dessen Metallanteil zu mehr als 90 % aus Cobalt bestand, und 540 ml Ammoniak (flüssig) dosiert. Anschließend wurden 5 bar Wasserstoff aufgepresst und der Autoklav verschlossen. Das Gemisch wurde unter Rühren für 8 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 80 % zum primären Amin ermittelt werden. Ein Gehalt an sek. und tert. Aminen war dabei nicht nachweisbar.

### Beispiel 8

In einen Rührautoklav mit 2 I Volumen wurden 1 kg Fettalkoholoxpropylat mit einer mittleren Molmasse von 310 g/mol, 50 g eines Trägerkatalysators, dessen Metallanteil zu mehr als 95 % aus Cobalt bestand, und 540 ml Ammoniak (flüssig) dosiert. Anschließend wurden 5 bar Wasserstoff aufgepresst und der Autoklav verschlossen. Das Gemisch wurde unter Rühren für 24 Stunden auf 190°C erwärmt. Nach dem Abkühlen wurde langsam entspannt um den Ammoniak und den Wasserstoff zu entfernen. Das verbleibende Reaktionsgemisch wurde im Vakuum von restlichen flüchtigen Anteilen befreit und filtriert. Über die Bestimmung der Aminzahl konnte ein Umsetzungsgrad von 75 % zum entsprechenden prim. Amin ermittelt werden. Der Gehalt an sek. und tert. Aminen war < 1 mol-%.

### Beispiel 9

Ein Festbettreaktor mit einem Durchmesser von 5 cm und einer Länge von 2,5 m wurde mit Tabletten eines Trägerkatalysators, dessen Metallanteil zu mehr als 90 % aus Cobalt bestand, befüllt und der Katalysator unter reduktiven Bedingungen aktiviert. Anschließend wurden bei einer Temperatur von 190°C und einem Druck von 100 bar 1 kg/h Fettalkoholpropoxylat mit einer mittleren Molmasse von 310 g/mol, 2,5 kg/h Ammoniak und 50 l/h Wasserstoff in den Reaktor eingespeist. Nachdem sich im Reaktor stabile Reaktionsverhältnisse ausgebildet hatten, wurden Proben des Produktaustrags genommen. Über die Bestimmung der Aminzahl konnte ein Umsetzungsgrad von größer als 98 % ermittelt werden.

### Beispiel 10

Es wurde wie in Beispiel 9 beschrieben ein Festbettreaktor mit Katalysator befüllt und konditioniert. Anschließend wurden bei einer Temperatur von 195°C und einem Druck von 190 bar 1 kg/h Fettalkoholpropoxylat mit einer mittleren Molmasse von 310 g/mol, 2,5 kg/h Ammoniak und 50 l/h Wasserstoff in den Reaktor eingespeist. Nachdem sich im Reaktor stabile Reaktionsverhältnisse ausgebildet hatten, wurden Proben des Produktaustrags genommen. Über die Bestimmung der Aminzahl konnte ein Umsetzungsgrad von größer als 95 % ermittelt werden.

### Beispiel 11

Es wurde wie in Beispiel 9 beschrieben ein Festbettreaktor mit Katalysator befüllt und konditioniert. Anschließend wurden bei einer Temperatur von 195°C und einem Druck von 190 bar 1 kg/h Methylpolyalkylenglykol mit einer mittleren Molmasse von 2000 g/mol, 1,5 kg/h Ammoniak und 100 l/h Wasserstoff in den Reaktor eingespeist. Nachdem sich im Reaktor stabile Reaktionsverhältnisse ausgebildet hatten, wurden Proben des Produktaustrags genommen. Über die Bestimmung der Aminzahl konnte ein Umsetzungsgrad von größer als 95 % ermittelt werden.

## Patentansprüche

1. Verfahren zur Herstellung von Aminen der Formel 1 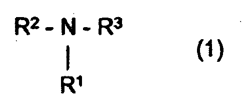 worin R² einen organischen Rest darstellt, der zwischen 2 und 600 Alkoxygruppen umfasst, und R¹ und R³ gleich oder verschieden sind und Wasserstoff oder einen organischen Rest mit 1 bis 400 Kohlenstoffatomen darstellen, indem man eine Verbindung der Formel 2 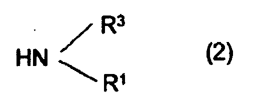 mit einer Verbindung der Formel 3
R²-OH
in Gegenwart von Wasserstoff mit einem Katalysator in Verbindung bringt, der metallhaltig ist und dessen Metallgehalt zu mindestens 95 Gew.-% aus Cobalt besteht.

2. Verfahren nach Anspruch 1, worin R¹ und R³ Wasserstoff, ein Alkylrest mit 1 bis 50 Kohlenstoffatomen, ein Alkenylrest mit 2 bis 50 Kohlenstoffatomen, ein Arylrest mit 6 bis 50 Kohlenstoffatomen, oder ein Alkylarylrest mit 7 bis 50 Kohlenstoffatomen sind.

3. Verfahren nach Anspruch 1 und/oder 2, worin R¹ und R³ der gleichen Definition wie R² entsprechen.

4. Verfahren nach einem oder mehreren der Ansprüche 1 bis 3, worin R¹ und R³ Aminogruppen enthalten.

5. Verfahren nach einem oder mehreren der Ansprüche 1 bis 4, worin R¹ und R³ der Formel 5 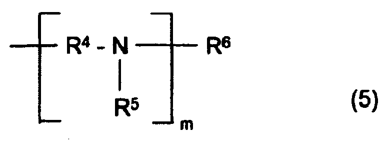 entsprechen, worin R⁴ eine zweiwertige Kohlenwasserstoffgruppe mit 1 bis 50 Kohlenstoffatomen und R⁵ und R⁶ jeweils Wasserstoff oder eine einwertige Kohlenwasserstoffgruppe mit 1 bis 50 Kohlenstoffatomen sein können, wobei jedes von R⁴, R⁵ und R⁶ 1 bis 200 Alkoxygruppen umfassen kann, und m für eine Zahl von 1 bis 10 steht.

6. Verfahren nach einem oder mehreren der Ansprüche 1 bis 5, worin das Amin der Formel (1) für ein Polyamin der Formel 11
R⁷(NHR⁸)ₙ (11)
steht, und worin R⁷ für einen n-wertigen organischen Rest mit 2 bis 400 Kohlenstoffatomen, R⁸ für einen Rest wie R¹, und n für eine ganze Zahl von 2 bis 20 steht.

7. Verfahren nach einem oder mehreren der Ansprüche 1 bis 6, worin die Reaktionstemperatur 50 bis 250°C beträgt.

8. Verfahren nach einem oder mehreren der Ansprüche 1 bis 7, worin der Druck 1 bis 300 bar beträgt.

## Claims

1. A process for preparing amines of the formula I 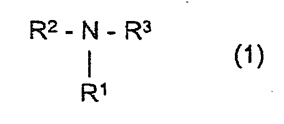 in which R² is an organic radical which comprises between 2 and 600 alkoxy groups, and R¹ and R³ are the same or different and are each hydrogen or an organic radical having from 1 to 400 carbon atoms, by combining a compound of the formula 2 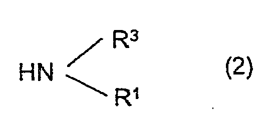 with a compound of the formula 3
R²-OH
in the presence of hydrogen with a catalyst which is metallic and whose metal content consists of cobalt to an extent of at least 95% by weight.

2. The process as claimed in claim 1, in which R¹ and R³ are each hydrogen, an alkyl radical having from 1 to 50 carbon atoms, an alkylene radical having from 2 to 50 carbon atoms, an aryl radical having from 6 to 50 carbon atoms or an alkylaryl radical having from 7 to 50 carbon atoms.

3. The process as claimed in claim 1 and/or 2, in which R¹ and R³ are each as defined for R².

4. The process as claimed in one or more of claims 1 to 3, in which R¹ and R³ each contain amino groups.

5. The process as claimed in one or more of claims 1 to 4, in which R¹ and R³ each correspond to the formula 5 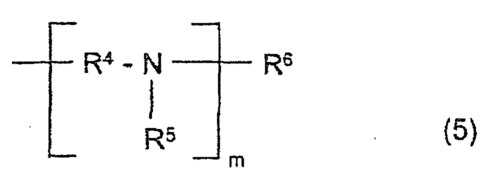 in which R⁴ is a divalent hydrocarbon group having from 1 to 50 carbon atoms and R⁵ and R⁶ may each be hydrogen or a monovalent hydrocarbon group having from 1 to 50 carbon atoms, where each of R⁴, R⁵ and R may comprise from 1 to 200 alkoxy groups, and m is from 1 to 10.

6. The process as claimed in one or more of claims 1 to 5, in which the amine of the formula (1) is a polyamine of the formula 11
R⁷(NHR⁸)ₙ (11)
and in which R⁷ is an n-valent organic radical having from 2 to 400 carbon atoms, R⁸ is a radical such as R¹, and n is an integer from 2 to 20.

7. The process as claimed in one or more of claims 1 to 7, in which the reaction temperature is from 50 to 250°C.

8. The process as claimed in one or more of claims 1 to 7, in which the pressure is from 1 to 300 bar.

## Revendications

1. Procédé pour la préparation d'amines de formule 1, 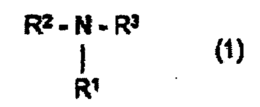 dans laquelle R² représente un radical organique, qui comprend entre 2 et 600 groupes alcoxy, et R¹ et R³ sont identiques ou différents et représentent hydrogène ou un radical organique comprenant 1 à 400 atomes de carbone, selon lequel on rassemble un composé de formule 2 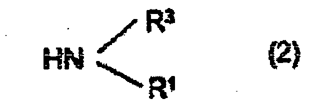 avec un composé de formule 3
R²-OH
en présence d'hydrogène avec un catalyseur, qui contient du métal et dont la teneur en métal est constituée à raison d'au moins 95% en poids de cobalt.

2. Procédé selon la revendication 1, dans lequel R¹ et R³ représentent hydrogène, un radical alkyle comprenant 1 à 50 atomes de carbone, un radical alcényle comprenant 2 à 50 atomes de carbone, un radical aryle comprenant 6 à 50 atomes de carbone ou un radical alkylaryle comprenant 7 à 50 atomes de carbone.

3. Procédé selon la revendication 1 et/ou 2, dans lequel R¹ et R³ ont la même signification que R².

4. Procédé selon l'une ou plusieurs des revendications 1 à 3, dans lequel R¹ et R³ contiennent des groupes amino.

5. Procédé selon l'une ou plusieurs des revendications 1 à 4, dans lequel R¹ et R³ correspondent à la formule 5 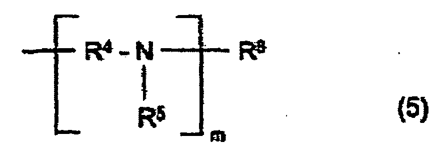 dans laquelle R⁴ peut représenter un groupe hydrocarboné divalent comprenant 1 à 50 atomes de carbone et R⁵ et R⁶ peuvent représenter à chaque fois hydrogène ou un groupe hydrocarboné monovalent comprenant 1 à 50 atomes de carbone, chacun parmi R⁴, R⁵ et R⁶ pouvant comprendre 1 à 200 groupes alcoxy et m représente un nombre de 1 à 10.

6. Procédé selon l'une ou plusieurs des revendications 1 à 5, dans lequel l'amine de formule (1) représente une polyamine de formule 11
R⁷(NHR⁸)ₙ (11)
dans laquelle R⁷ représente un radical organique n-valent comprenant 2 à 400 atomes de carbone, R⁸ représente un radical tel que R¹ et n représente un nombre entier de 2 à 20.

7. Procédé selon l'une ou plusieurs des revendications 1 à 6, dans lequel la température de réaction est de 50 à 250°C.

8. Procédé selon l'une ou plusieurs des revendications 1 à 7, dans lequel la pression de réaction est de 1 à 300 bars.
